# EUROPEAN PATENT APPLICATION

(11) **EP 4 498 230 A1**
(43) Date of publication of application: **29.01.2025**
(21) Application number: 23188514.6
(22) Date of filing: 28.07.2023
(51) Int. Cl.: G06F 8/38, A61B 5/18

(54) **METHOD FOR EVALUATING RELEVANCE OF AN ALTERABLE ATTRIBUTE OF AN ELEMENT OF A DESIGN FEATURE**

(71) Applicant: Rimac Technology LLC, 10431 Sveta Nedelja (HR)
(72) Inventor: PHILIP, Jaic Jacob, 10431 Sveta Nedelja (HR); HADZIABDIC, Haris, 71000 Sarajevo (BA); AWASTHI, Abhishek, 10000 Zagreb (HR)
(74) Representative: Penza, Giancarlo

(57) **Abstract**

A method is described for evaluating relevance of an alterable attribute of an element of a design feature. The method includes processing a plurality of sensory responses and generating therefrom a baseline response of the subject, includes receiving data indicative of the attribute of the element of the design feature, includes generating a plurality of synchronized sensory responses of the subject associated with said attribute, includes generating cognitive data of the subject associated with the attribute of the element, and includes comparing the cognitive data with respect to an attribute threshold index associated with the attribute of the element in order to ascertain whether a value of the cognitive data is above the attribute threshold index.

## Description

### TECHNICAL FIELD OF THE INVENTION

The present invention generally relates to the field of improvement of user experience design.

More in particular, the invention concerns the evaluation of relevance of an alterable attribute of an element of a design feature.

### BACKGROUND OF THE INVENTION

User experience design (also referred as "UX design") is the process of defining the experience a user would go through when interacting with a product, system or service.

Design decisions are often driven by research to understand who their target users are and what the user's needs are; design decisions are also driven by data analysis and test results.

Automatic evaluation methods are also known for product experience.

CN1 12541668 A discloses an automatic method and system for product experience, using an electroencephalogram signal and eye movement signals and a suitable processing of the signals for calculating visual attention information of each region of interest of the tested product and a visual-related experience emotion index value of the subject during an attention period of each region of interest of the product.

The Applicant has perceived that a disadvantage of this prior art is that the product design may not provide a good experience for the user, who may be frustrated; moreover, it does not provide sufficient suggestions about how to modify the product design in order to improve its adaptability.

### SUMMARY OF THE INVENTION

The present invention relates to a method for evaluating relevance of an alterable attribute of an element of a design feature as defined in the enclosed claim 1 and by its preferred embodiments disclosed in the dependent claims 2 to 15.

The basic idea is to identify a baseline response of the subject by removing any bias of the subject and to evaluate cognitive data of the subject associated with an attribute of an element of a design feature taking into account values of sensory responses above the baseline response.

The Applicant has perceived that the evaluation method according to the present invention has the advantage of optimizing the arrangement of an attribute of an element of a design feature, so that the intention of the user is met (i.e., he/she is not frustrated) when interacting with the product, graphic user interface, system or service.

### BRIEF DESCRIPTION OF THE DRAWINGS

Figure 1 schematically shows a block diagram of an electronic system to evaluate relevance of an alterable attribute of an element of a design feature according to the invention.
Figures 2A-2B show a flow diagram of a method for evaluating relevance of an alterable attribute of an element of a design feature according to the invention.

### BEST MODE FOR CARRYING OUT THE INVENTION

It should be noted that in the description that follows identical or similar blocks, components or modules have the same reference numerals, regardless of whether they are shown in different embodiments of the invention.

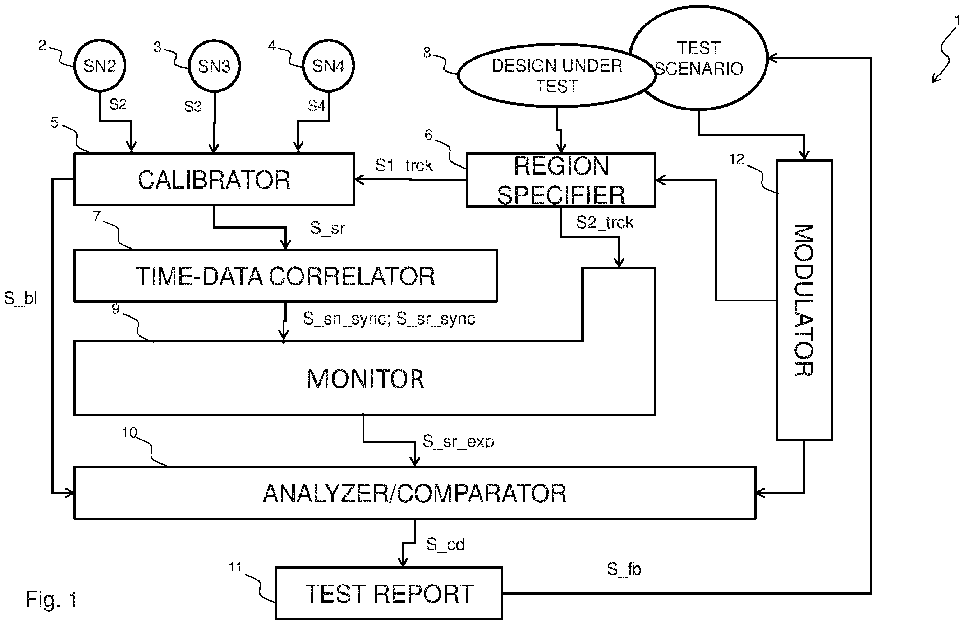

Referring to Figure 1, it shows a block diagram of an electronic system 1 to evaluate relevance of an alterable attribute of an element of a design feature according to the invention.

The electronic system 1 may be used for testing an attribute of an element of a design feature according to the following examples:
- the element is a virtual button (i.e. icon) on a graphic user interface, such as a textual or graphical indicator on a dashboard of a vehicle (in particular, a motor vehicle) for informing the driver about a remaining fuel range or a remaining electric power range: in this case the attribute may be the position or colour or size or texture of the virtual button (e.g., the position of the textual or graphical indicator on the dashboard);
- the element is a physical button on an object, such as a switch button integrated in a door of a building for unlocking the opening of the door or the switch button is integrated in a body of a vehicle: in this case the attribute may be the position of the physical button (e.g. the position of the switch button on a structure of the door or on the body of the vehicle);
- the element is an opening/closing handle on a door to access a limited environment or a vehicle: in this case, the attribute may be the position of the opening/closing handle of the door;
- the element is a tool, knob or other physical instrument used to operate or perform operations on an industry line or inside a vehicle: in this case the attribute may be reach and access proximity that determines the best natural position for a control based on a real-life scenario it is being used.

The electronic system 1 includes three sensory units 2, 3, 4 associated with a subject for carrying out a test of a design feature of a product, graphic user interface, system or service.

The term "sensory unit" means that it includes one or more sensors adapted to generate one or more respective sensory signals (in particular, analog or digital signals), wherein the sensory unit is associated with the subject who may be a human being or it can be a robot.

The sensory units 2, 3, 4 may include one or more of:
- an eye tracking camera;
- an electrocardiography sensor;
- an electroencephalography (EEG) sensor;
- a microphone;
- a touch point tracker;
- a taste sensor;
- a motion detector.

In particular:
- when a sensory unit is an eye tracking camera placed in front of the subject face, the sensory signal is one or more electric signals indicating the position of the eyes of the subject and the sensory response may be the point of gaze (i.e. position) of the eyes of the subject;
- when a sensory unit is an electrocardiography sensor, the sensory signals are a plurality of analog signals generated by the electrodes attached to the body of the subject and the sensory response may be the heartbeat (for example, resting heart rate) generated as a function of the plurality of analog signals of the electrodes;
- when a sensory unit is an electroencephalography sensor, the sensory signals are a plurality of analog signals generated by the electrodes attached to the scalp of the subject and the sensory response may be one or more signals generated as a function of the plurality of analog signals of the electrodes, such as EEG and brain activity indicative of facial expression associated to an emotion, or EEG and brain activity indicative of the cognitive process involved in an action by the subject;

- when a sensory unit is a microphone positioned close to the mouth of the subject, the sensory signal is an analog signal indicating a recording of the voice of the subject and the sensory response may be a digital voiceprint generated as a function of the voice recording, such as verbal responses and natural language processing;
- when a sensory unit is a touch point tracker associated with a hand of the subject, the sensory signal is an analog signal indicating touch perceptions and the sensory responses may be indicative emotion;
- when a sensory unit is a taste sensor associated with a hand of the subject, the sensory signal is touch movements and the sensory responses may be digital or analog responses corresponding to the movements;
- when a sensory unit is a motion detector (for example, a camera) positioned inside an environment wherein the subject is placed, the sensory signal is an electric signal (for example, a video stream) indicative of a detected movement of the subject inside the environment and the sensory response may be data indicative of the position of the subject inside the environment.

For the purpose of explaining the invention, Figure 1 shows three sensory units 2, 3, 4, but more in general the electronic system 1 may include one or more sensory units, each one including one or more sensors adapted to generate one or more corresponding sensory signals, such that at least two sensory signals are generated by the one or more sensory units.

The electronic system 1 further includes the following blocks:
- a Calibrator 5;
- a Time-data Correlator 7;
- a Monitor 9;
- an Analyzer/Comparator 10;
- a Test report 11;
- a Region specifier 6;
- a Modulator 12.

The group composed of the Calibrator 5, Time-data Correlator 7, Monitor 9 and Analyzer/Comparator 10 may be implemented by means of a software program running on a processing unit, such as a microprocessor.

Alternatively, the groups composed of the Calibrator 5, time-data Correlator 7, Monitor 9 and Analyzer/Comparator 10 may be implemented at least partially with hardware devices.

A design under test may be affected by noise generated by the environment wherein the design is placed, so that a plurality of pre-calibrated regions of interest in the design may be provided and the elements of the design feature under test may be positioned inside said plurality of region of interests.

A region of interest may be defined by placing a respective sensor on the design, such that the attribute (or changed attribute) of the element of the design feature is positioned within the region of interest.

The sensor may be a readable tag, such as a Quick Response (QR) code. Therefore the Region specifier 6 has the function of generating a first tracking signal S1_trck for notifying the Calibrator 5 whether current measurements of the one or more sensory units 2, 3, 4 are valid or invalid.

Moreover, the Region specifier 6 has the function of generating a second tracking signal S2_trck for notifying the Monitor 9 whether current measurements of the sensory units 2, 3, 4 are valid or invalid and (when valid) also for indicating the specific region of interest, out of the plurality of region of interests, wherein the element of the design feature is positioned, so that data received from the sensory units 2, 3, 4 is correlated with the region which the subject is interacting with.

In one embodiment, an external region of interest may also be defined outside a region of interest and the electronic system 1 is capable to determine that an attribute (or a changed attribute) of the element of the design feature may also be placed within the external region of interest.

The Calibrator 5 is connected at the input to the sensory units 2, 3, 4 and to the Region specifier 6 and it is connected at the output to the Time-data Correlator 7 and to the Analyzer/Comparator 10.

The Calibrator 5 is configured to receive three sensory signals S2, S3, S4 generated by the sensory units 2, 3, 4, respectively, and it is configured to receive from the Region specifier 6, the first tracking signal S1_trck indicating whether current measurements of the sensory signals S2, S3, S4 are valid or invalid.

The Calibrator 5 has the function of generating three sensory responses S_sr of the subject, as a function of the three sensory signals S2, S3, S4 generated by the sensory units 2, 3, 4, respectively.

Moreover, the Calibrator 5 has the function of generating a baseline response S_bl of the subject taking into account the three sensory responses S_sr of the subject, by removing any bias in the sensory responses of the subject who is going to carry out a test of an attribute of an element of a design feature.

For example, suppose that the sensory unit 2 is an electrocardiography sensor and one of the corresponding sensory responses of the subject is the heartbeat.

In this example, it may occur that a human subject carries out the test when being excited for any particular reason, so that heartbeat of the subject is higher than in a normal condition.

In this case, it is necessary to filter the values of the sensory response signal indicating the heartbeat of the subject, by generating a baseline response signal of the heartbeat of the subject, so that the test may take into account only the difference of the values of the heartbeat signal generated when carrying out the test of the attribute with respect to the baseline response: in this way the test is not affected by different states of different human subjects.

The time-data Correlator 7 is connected at the input to the Calibrator 5 and it is connected at the output to the Monitor 9.

The Time-data Correlator 7 has the function of synchronizing each of the plurality of sensory signals and the plurality of sensory responses.

In fact, the plurality of sensory signals may be generated by different sensory units, each one having its internal clock signal for generating the sensory signals.

The Time-data Correlator 7 performs alignment of the different sensory signals with a common clock signal and performs alignment of the different sensory responses with a common clock signal.

Therefore, the output of the time-data Correlator 7 is a plurality of synchronized sensory signals S_sn_sycn and a plurality of synchronized sensory responses S_sr_sycn associated with the subject, either when the subject is not yet exposed to the design or after that the subject is exposed to the design.

The Monitor 9 is connected at the input to the Time-data Correlator 7 and to the Region specifier 6 and it is connected at the output to the Analyzer/Comparator 10.

The Monitor 9 has the function of collecting, from the selected region of interest according to the value of the second tracking signal S2_trck, the plurality of synchronized sensory signals S_sn_sync after the subject is exposed to the design and of generating therefrom a further plurality of sensory responses S_sr_exp after the subject is exposed to the design.

Therefore, the output of the Monitor 9 is the further plurality of sensory responses S_sr_exp after the subject is exposed to the design.

The Analyzer/Comparator 10 is connected at the input to the Monitor 9, to the Calibrator 5 and to the Modulator 12 and it is connected at the output to the Test report 11.

The Analyzer/Comparator 10 has the function of generating cognitive data S_cd of the subject associated with the considered attribute of the element of the design feature, taking into account the baseline response S_bl and the plurality of synchronized sensory responses S_sr_exp after the subject is exposed to the design, as it will be explained more in detail afterwards.

The Test report 11 has the function of generating a textual or graphical representation of the cognitive data S_cd and it includes information for generating suggestions about how to change the actual attribute of the element of the design feature under test.

In particular, the Test report 11 is configured to generate a feedback signal S_fb carrying assigned data indicative of a changed attribute of the element of the design feature under test, wherein said data indicative of a changed attribute may be assigned by means of a human person or it may be automatically generated by means of the software program running the method of the flow diagram 100.

In one embodiment, the feedback signal S_fb is generated based on a heatmap or a textual report generated as a function of the plurality of sensory responses of the subject.

For example, the heatmap shows the time spent per unit area of a design feature, a user journey report is generated as a function of the gaze path of the user, and cognitive data together representing the visual hierarchy of the different elements in the design feature, graphs indicating the plurality of the processed sensory responses against the synchronized time and a summary data report that compares the attribute threshold index against the index calculated from the cognitive data.

The Modulator 12 is connected at the input to a test scenario and it is connected at the output to the Region specifier 6 and to the Analyzer/Comparator 10.

The Modulator 12 has the function of controlling to actuate the action the user wishes to perform.

For example, the user wants to select a setting, the Modulator 12 ensures the user has selected the right setting to proceed with the remaining data capture.

Therefore, the Modulator 12 is a controller for a given test in order not to deter to a different scenario than the one in test.

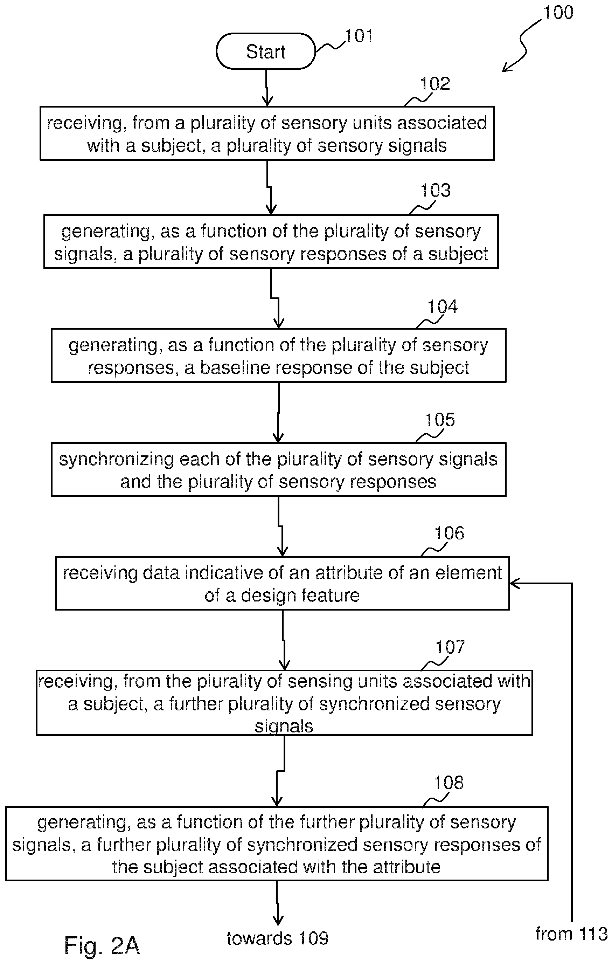

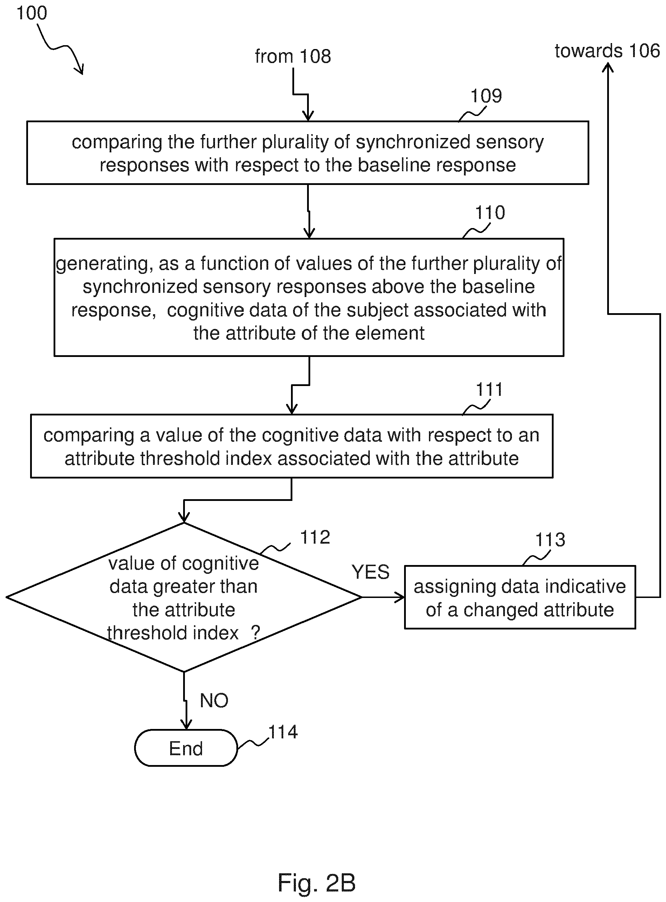

Referring to Figures 2A-2B which show a flow diagram 100 of the method for evaluating relevance of an alterable attribute of an element of a design feature.

The steps of the flow diagram 100 are implemented by means of a software program running on a processing unit (for example, a microprocessor) of the electronic system 1.

In particular, steps 102, 103, 104 are executed in Calibrator block 5, step 105 is executed in Time-data Correlator block 7, steps 106, 107, 108 are executed in Monitor block 9, steps 109, 110, 111, 112 are executed in Analyzer/Comparator block 10 and step 113 is executed in Test report 11 and in Modulator block 12.

The flow diagram starts with step 101.

From step 101 the flow diagram proceeds with step 102 of receiving from a plurality of sensory units associated with a subject, a plurality of sensory signals (for example, analog or digital signals), wherein the subject is not yet exposed to the design under test.

From step 102 the flow diagram proceeds with step 103 of generating, as a function of the plurality of sensory signals, a plurality of sensory responses of a subject.

From step 103 the flow diagram proceeds with step 104 of generating, as a function of the plurality of sensory responses, a baseline response of the subject.

From step 104 the flow diagram proceeds with step 105 of synchronizing each of the plurality of sensory signals and the plurality of sensory responses.

In one embodiment, in step 104 the baseline response is generated using multiple steps, by means of a standardized instruction set to form a moving average value.

For example, the standardized instruction set is black dot on a screen that moves and the instruction for the subject is to visually follow the black dot where in the gaze signal will be moving around the dot which is averaged to match the position of the dot henceforth further used to track eye position.

From step 105 the flow diagram proceeds with step 106 of receiving data indicative of an attribute of an element of a design feature.

From step 106 the flow diagram proceeds with step 107 of receiving from the plurality of sensory units associated with a subject, a further plurality of synchronized sensory signals, after assigning said data indicative of the changed attribute.

From step 107 the flow diagram proceeds with step 108 of generating, as a function of the further plurality of sensory signals, a further plurality of synchronized sensory responses of the subject associated with the attribute.

From step 108 the flow diagram proceeds with step 109 of comparing the further plurality of synchronized sensory responses with respect to the baseline response.

From step 109 the flow diagram proceeds with step 110 of generating, as a function of the values of the further plurality of synchronized sensory responses (associated with said attribute) above the baseline response, cognitive data of the subject associated with the attribute of the element.

In other words, steps 109 and 110 define that, for each time instant, only the respective values of the further plurality of synchronized sensory responses (associated with the considered attribute) which are above the baseline response values at the corresponding time instant are taken into account for generating the cognitive data of the subject.

From step 110 the flow diagram proceeds with step 111 comparing the cognitive data with respect to an attribute threshold index associated with the attribute.

From step 111 the flow diagram proceeds with step 112 wherein it is verified whether a value of the cognitive data is greater than the attribute threshold index:
- in case of positive answer, the flow diagram proceeds with step 113;
- in case of negative answer, the flow diagram proceeds with step 114 wherein it terminates.

The following are some examples of cognitive data and attribute threshold index:
- if the element is a textual or graphical indicator on a graphic user interface and the attribute is the position of the textual or graphical indicator, the cognitive data is the time and/or mental load indicative of anxiety required by the subject for detecting activation of the textual or graphical indicator and the attribute threshold index is the minimum acceptable time to visually detect the textual or graphical indicator by the subject;
- if the element is a switch button integrated into a door of a building or of a body of a vehicle (for example, a motor vehicle) and the attribute is the position of the switch button on a structure of the door or on the body of the vehicle, the cognitive data is the time and/or mental load indicative of convenience required by the subject for pushing the switch button to open/close the door and the attribute threshold index is the minimum acceptable time to push the switch button by the subject;
- if the element is a tool, knob or other physical instrument used to operate or perform operations on an industry line or inside a vehicle (for example, a motor vehicle) and the attribute is reach and access proximity that determines the best natural position for a control based on a real-life scenario it is being used, the cognitive data is the time, placement and mental load indicative of stress requirement by the subject for carrying of the intended operation and the attribute threshold index is the minimum acceptable time to visually identify and access the instrument by the subject.

In step 113 it is assigned data indicative of a changed attribute.

In one embodiment, step 113 further includes determining said data indicative of the changed attribute based on a heatmap or a textual report, which is generated as a function of the plurality of sensory responses of the subject.

The following are some examples of changing an attribute:
- changing the position of the textual or graphical indicator on a graphic user interface;
- changing the position of a virtual button on a screen;
- changing the position of the switch button on a structure of a door or on the body of a vehicle (for example, a motorvehicle);
- changing a colour or intensity (for example, from light to dark colour, or vice versa) of an element based on the reaction of the subject, so that the new color or intensity of the element has more effect on the subject;
- changing frequency or loudness of a voice (element);
- increasing or decreasing the flickering frequency of a light indicator, in order to gain more attention from the subject;
- changing the font size of a command (element).

More in general, one or more parameters of the attribute may be changed based on the required effect that it has on the subject, wherein it can be a predetermined list of parameters values (such as colours and/or sounds) of the attribute, possibly according to an order of effectiveness.

Changing a colour (from light to dark colour based on subject reaction, and required, e.g., if the response indicates too calm user, the colour can be changed to have more effect (be more appealing) to the user).

Likewise, voice (frequency or loudness), frequency of flickering of an indication light (increase frequency to gain more attention), font size of a command.

Basically all the parameters that can be changed based on the required effect that they have on the user (it can be a predetermined list of colour, sounds, and their order of effectiveness).

From step 113 the flow diagram returns to step 106 and the cycle composed of the steps 106, 107, 108, 109, 110, 111, 112 is repeated again for the changed attribute of the considered element of the design feature.

Therefore the cycle composed of steps 106, 107, 108, 109, 110, 111, 112 is repeated one or more times, till when the value of the cognitive data is not smaller than the value of the attribute threshold index: when this occurs, it is achieved an optimized arrangement of the attribute of the considered element of the design feature.

Alternatively, from step 113 the flow diagram returns to step 105 so that the synchronization of the plurality of sensory responses is repeated again and the cycle composed of the steps 105, 106, 107, 108, 109, 110, 111, 112 is repeated again for the changed attribute of the considered element of the design feature.

In one embodiment, the method for evaluating relevance of an alterable attribute of an element of a design feature further comprises the step of obtaining personal information pertaining to the subject, the personal information including one or more of demographic data, age, gender, ethnicity, region, country, economic status, education level, comprises the step of determining tags to be associated with the subject and comprises the step of adjusting the attribute threshold index based on the tags associated with the subject.

In fact, a subject who is used to reading Arabic script is likely to have a different response to any readable instructions based on whether its written left to right, or right to left; the method is able to determine such preferences of the subject and the attribute threshold index may be adjusted accordingly.

In one embodiment, upon determining personal information pertaining to multiple subjects, and upon performing the steps 102, 103, 104, 105, 106, 107, 108, 109, 110, 111, 112 with such multiple steps, the method for evaluating relevance of an alterable attribute of an element of a design feature includes the step of determining behavioral grouping, and it includes the step of recommending trends based on the behavioral grouping of demographic data to further filter recommendations.

Alternatively, in one embodiment, upon determining personal information pertaining to multiple subjects, and upon performing the steps 102, 103, 104, 105, 106, 107, 108, 109, 110, 111, 112 with such multiple steps, the method for evaluating relevance of an alterable attribute of an element of a design feature includes adjusting the attribute threshold index based on the tags associated with the multiple subjects.

In one embodiment, the method for evaluating relevance of an alterable attribute of an element of a design feature further includes the step of obtaining personal information pertaining to a new subject, the personal information including one or more of demographic data, age, gender, ethnicity, region, country, economic status, education level, it includes the step of determining tags and behavioral grouping associated with the new subject, and it includes the step of suggesting a changed attribute of the element of the design feature based on the determined tags and behavioral grouping associated with the new subject.

In fact, based on the demographic data, after performing multiple tests, the method for evaluating relevance of an alterable attribute of an element of a design feature will be able to automatically recommend trends based on the grouping of demographic data to further filter recommendations.

Moreover, the method may automatically detect demographics or tags, and may generate/modify tests and recommendations based on the demographic.

Further, the method for evaluating relevance of an alterable attribute of an element of a design feature will generate a model based on the sensory responses, after performing multiple tests, to predict user behavior for further design iterations: this helps basically in rapid prototyping, where newer design iterations can be evaluated for its success ratio before a live test.

In one embodiment, the method for evaluating relevance of an alterable attribute of an element of a design feature further includes the step of comparing the changed attribute of the element of the design feature with existing regulatory requirements (such as homologation/safety requirements), and it includes the step of affecting the changed attribute of the element of the design feature such that the existing regulatory requirements are complied.

In one embodiment, the method for evaluating the relevance of an alterable attribute of an element of a design feature further includes, before step 106, the step of defining a region of interest by means of at least one sensor (in particular at least one readable tag, such as QR codes), and includes the step of identifying the region of interest such that the attribute (or changed attribute) of the element of the design feature is positioned within the region of interest.

In one embodiment, the method for evaluating relevance of an alterable attribute of an element of a design feature further includes the step of identifying an external region of interest which is outside the region of interest, such that the attribute (or changed attribute) of the element of the design feature is positioned within the external region of interest.

For example, let's suppose that the attribute is the placement of a display of a navigation system inside the cockpit of a car. In this case the region of interest is the central console of the car dashboard, and the method is capable of determining that the display of the navigation system may also be placed outside the car dashboard, such as for example a head-up display.

It will be described hereinafter a first operation of electronic system 1, referring also to Figures 1 and 2A-2B.

It is supposed that a test is carried out for evaluating the position of a graphical indicator in a car dashboard for informing the driver about the remaining fuel range or electric power range of the car, for example, measured in kilometers (km), by measuring the time taken by the subject gaze (i.e. the cognitive data of the subject) for detecting the activation (i.e. switch on) of the graphical indicator of the remaining fuel/power range.

A human subject is placed in front of a screen of a desktop or laptop personal computer, wherein the screen has an appearance similar to that of a car dashboard: in this case, the electronic system 1 is implemented with the microprocessor of the personal computer running a software program performing the method of the flow diagram 100.

Moreover, the personal computer is equipped with a camera framing the face of the subject, in particular the eyes.

It is supposed that a maximum acceptable value of the time of the subject gaze for detecting the activation of the graphical indicator of the remaining fuel/power range is equal to 1 second (i.e., the attribute threshold index).

It is supposed that in a first iteration the graphical indicator of the remaining fuel/power range is positioned in a top-right area of the dashboard screen.

At time t0 the graphical indicator of the remaining fuel/power range is switched off, i.e., it is not visible by the subject on the dashboard screen.

At subsequent time t1 the graphical indicator of the remaining fuel/power range is switched on in the top-right area, for example indicating the value of 50 km: the microprocessor of the personal computer carries out the steps of the method of the flow diagram 100.

In particular, between times t1 and t2 the camera of the personal computer tracks the movement of the eyes of the subject (step 102 of the flow diagram 100), till when at time t2 the subject focuses his gaze on the switched-on graphical indicator in the top-right position and it is measured the time (cognitive data) taken by the subject for detecting the switch-on of the graphical indicator (steps 106, 107, 108, 109, 110 carried out on the microprocessor of the personal computer).

It is supposed that time (cognitive data) taken by the subject for detecting the switch-on of the graphical indicator is equal to 3 seconds (i.e., the time period comprised between t1 and t2), thus it is greater than the acceptable value (attribute threshold index) equal to 1 second (steps 111 and 112 of the flow diagram 100, option YES).

At subsequent time t3 the graphical indicator is switched off and the first iteration of the test terminates.

At subsequent time t4 the second iteration of the test starts: the software program running on the microprocessor of the personal computer controls (automatically or by means of input command from the subject or other users) the assignment of another position of the graphical indicator of the remaining fuel/power range (step 113 of the flow diagram 100) on the screen of the personal computer and it is supposed that the graphical indicator of the remaining fuel/power range is positioned in a bottom-left area of the dashboard screen.

At subsequent time t6 the graphical indicator of the remaining fuel/power range is switched on in the bottom-left area, for example indicating the value of 40 Km: the microprocessor of the personal computer carries out again the steps 103, 104, 105, 106, 107, 108, 109, 110 of the method of the flow diagram 100.

In particular, between times t6 and t7 the camera of the personal computer tracks the movement of the eyes of the subject (step 102 of the flow diagram 100) till when at time t7 the subject focuses his gaze on the switched-on graphical indicator in the bottom-left position and it is measured again the time (cognitive data) taken by the subject for detecting the switch-on of the graphical indicator (steps 106, 107, 108, 109, 110).

It is supposed that time (cognitive data) taken by the subject for detecting the switch-on of the graphical indicator is equal to 2 seconds (i.e., the time period between t6 and t7), thus it is again greater than the acceptable value (attribute threshold index) equal to 1 second (steps 111 and 112 of the flow diagram 100, option YES).

At time t9 the graphical indicator is switched off again and the second iteration of the test terminates.

At subsequent time t10 the third iteration of the test starts: the software program running on the microprocessor of the personal computer controls (automatically or by means of input command from the subject or other users) the assignment of another position of the graphical indicator of the remaining fuel/power range (step 113 of the flow diagram 100) on the screen of the personal computer and it is supposed that the graphical indicator of the remaining fuel/power range is positioned in a central area of the dashboard screen.

At subsequent time t11 the graphical indicator of the remaining fuel/power range is switched on in the bottom-left area, for example indicating the value of 30 Km: the microprocessor of the personal computer carries out the steps of the method of the flow diagram 100.

In particular, between times t11 and t12 the camera of the personal computer tracks the movement of the eyes of the subject (step 102 of the flow diagram 100) till when at time t7 the subject focuses his gaze on the switched-on graphical indicator in the bottom-left position and it is measured again the time (cognitive data) taken by the subject for detecting the switch-on of the graphical indicator (steps 106, 107, 108, 109, 110).

It is supposed that time (cognitive data) taken by the subject for detecting the switch-on of the graphical indicator is equal to 0.5 seconds (i.e. the time period between t11 and t12 is 0.5 seconds), thus it is smaller than the acceptable value (attribute threshold index) equal to 1 second (steps 111 and 112 of the flow diagram 100, option NO).

Therefore the test terminates after the third iteration (step 114) and the outcome is that the best arrangement of the position of a graphical indicator on a car dashboard for informing the driver about a remaining fuel or power range of the car is in the center of the car dashboard.

It will be described hereinafter a second operation of electronic system 1, referring also to Figures 1 and 2A-2B.

It is supposed that a test is carried out for evaluating the position of an opening/closing handle (or an opening/closing button) placed on a car door.

It is supposed that a maximum acceptable value of the time for the subject to catch with the hand the handle of the car door (or to touch with a finger the button on the car door) is equal to 5 seconds, when the human subject is positioned in front of the car door.

A human subject is positioned in front of a touch screen displaying an image representing a car door with a virtual button for opening/closing the car door.

Moreover, the screen is connected to a personal computer running a software program performing the method of the flow diagram 100.

It is supposed that in a first iteration the virtual button is placed in a top position of the image on the screen representing the car door.

At time t10 the virtual button is switched off, i.e., it is not visible by the subject on the screen.

At subsequent time t11 the virtual button is switched on in the top position of the image representing the car door (for example, the virtual button is a steady green light having a circular shape): the microprocessor of the personal computer carries out the steps of the method of the flow diagram 100.

In particular, between times t11 and t12 the subject moves his hand till when at time t12 a finger of the subject touches the virtual button on the screen and it is measured the time (cognitive data) taken by the subject for touching the virtual button (steps 106, 107, 108, 109, 110 carried out on the microprocessor of the personal computer).

It is supposed that time (cognitive data) taken by the subject for touching the virtual button is equal to 10 seconds (i.e., the time period comprised between times t11 and t12), thus it is greater than the acceptable value (attribute threshold index) equal to 5 seconds (steps 111 and 112 of the flow diagram 100, option YES).

At subsequent time t13 the virtual button is switched off and the first iteration of the test terminates.

At subsequent time t14 the second iteration of the test starts: the software program running on the microprocessor of the personal computer controls (automatically or by means of input command from the subject or other users) the assignment of another position of the virtual button (step 113 of the flow diagram 100) in the image of the screen representing the car door and it is supposed that the virtual button is positioned in a central area of the image representing the car door.

At subsequent time t16 the virtual button is switched on in the central position of the image representing the car door (in the example, the virtual button is a steady green light having a circular shape): the microprocessor of the personal computer carries out again the steps 103, 104, 105, 106, 107, 108, 109, 110 of the method of the flow diagram 100.

In particular, between times t16 and t17 the subject moves his hand till when at time t17 a finger of the subject touches the virtual button on the screen and it is measured the time (cognitive data) taken by the subject for touching the virtual button (steps 106, 107, 108, 109, 110 carried out on the microprocessor of the personal computer).

It is supposed that time (cognitive data) taken by the subject for touching the virtual button is equal to 4 seconds (i.e. the time period comprised between t16 and t17), thus it is smaller than the acceptable value (attribute threshold index) equal to 5 seconds (steps 111 and 112 of the flow diagram 100, option NO).

Therefore the test terminates after the second iteration (step 114) and the outcome is that the best arrangement of the position of a virtual button (or a handle) on a car door for opening/closing the door is in the center of the car door.

It is also an object of the present invention a non-transitory computer-readable storage medium comprising instructions which, when executed by a computer, cause the computer to carry out the steps 104, 105, 106, 107, 108, 109, 110, 111, 112 and 113 of the method for evaluating relevance of an alterable attribute of an element of a design feature as above explained.

It is also an object of the present invention an electronic system 1 to evaluate relevance of an alterable attribute of an element of a design feature, the system comprising at least one sensory unit associated with a subject, the at least one sensory units being configured to generate a plurality of sensory signals S2, S3, S4.

The electronic system 1 further comprises a processing unit (such as a microprocessor) carrying out the functionalities of the Calibrator 5, Time-data Correlator 7, Monitor 9 and Analyzer/comparator 10.

The processing unit is configured to receive the plurality of sensory signals and generate therefrom a plurality of sensory responses of the subject, is configured to process the plurality of sensory responses and generate therefrom a baseline response of the subject, is configured to synchronize each of the plurality of sensory signals and the plurality of sensory responses, is configured to receive data indicative of the attribute of the element of the design feature, is configured, after reception of said data indicative of the attribute, to receive, from the plurality of sensory units, a further plurality of synchronized sensory signals and generate therefrom a further plurality of synchronized sensory responses of the subject associated with said attribute, is configured to compare the further plurality of sensory responses with respect to the baseline response and generate, as a function of values of the further plurality of synchronized sensory responses above the baseline response, cognitive data of the subject associated with the attribute of the element, and it is configured to compare the cognitive data with respect to an attribute threshold index associated with the attribute of the element to ascertain whether a value of the cognitive data is above the attribute threshold index.

In one embodiment, the processing unit is further configured to detect that said value of the cognitive data is above the attribute threshold index and assign data indicative of a changed attribute of the element of the design feature, is configured, after the assignment of said data indicative of the changed attribute, to receive, from at least one sensory unit associated with the subject, a further plurality of sensory signals and generate therefrom a further plurality of synchronized sensory responses of the subject associated with said changed attribute, is configured to compare the further plurality of synchronized sensory responses with respect to the baseline response and generate, as a function of values of the further plurality of synchronized sensory responses above the baseline response, further cognitive data of the subject associated with the changed attribute of the element, and is configured to compare the further cognitive data with respect to an attribute threshold index associated with the changed attribute to determine if the further cognitive data is above the attribute threshold index.

## Claims

1. A computer-implemented method (100) for evaluating relevance of an alterable attribute of an element of a design feature, the method comprising:
a) receiving (102) from at least one sensory unit associated with a subject, a plurality of sensory signals (S2, S3, S4) and generating (103) therefrom a plurality of sensory responses (S_sr) of the subject;
b) processing (104) the plurality of sensory responses and generating therefrom a baseline response of the subject;
c) synchronizing (105) each of the plurality of sensory signals and the plurality of sensory responses;
d) receiving (106) data indicative of the attribute of the element of the design feature;
e) after receiving said data indicative of the attribute, receiving (107), from the at least one sensory unit, a further plurality of synchronized sensory signals and generating (108) therefrom a further plurality of synchronized sensory responses of the subject associated with said attribute;
f) comparing (109) the further plurality of synchronized sensory responses with respect to the baseline response and generating (110), as a function of values of the further plurality of synchronized sensory responses above the baseline response, cognitive data (S_cd) of the subject associated with the attribute of the element; and
g) comparing (111, 112) the cognitive data with respect to an attribute threshold index associated with the attribute of the element in order to ascertain whether a value of the cognitive data is above the attribute threshold index.

2. The method as claimed in claim 1, further comprising:
h) detecting (112) that said value of the cognitive data is above the attribute threshold index and assigning (113) data indicative of a changed attribute of the element of the design feature;
i) after assigning said data indicative of the changed attribute, receiving (107), from the at least one sensory unit associated with the subject, a further plurality of sensory signals and generating (108) therefrom a further plurality of synchronized sensory responses of the subject associated with said changed attribute;
j) comparing (109) the further plurality of synchronized sensory responses with respect to the baseline response and generating, as a function of values of the further plurality of synchronized sensory responses above the baseline response, further cognitive data of the subject associated with the changed attribute of the element; and
k) comparing (111, 112) the further cognitive data with respect to an attribute threshold index associated with the changed attribute to determine if the further cognitive data is above the attribute threshold index.

3. The method of claim 2, further including repeating at least one time steps h), i), j), k) with the changed attribute, so as to generate further cognitive data, till when the further cognitive data is not smaller than the attribute threshold index.

4. The method of any of the previous claims, wherein in step b) the baseline response is generated by means of a standardized instruction set to form a moving average value.

5. The method of any of the previous claims, wherein in step k) the attribute threshold index is determined by means of a pre-notion expectation of the subject and by refining using the comparison result of step g).

6. The method of claims 2 to 5, wherein step h) further includes determining said data indicative of the changed attribute of the element of the design feature based on a heatmap or textual report generated as a function of the plurality of sensory responses of the subject.

7. The method of any of the previous claims, wherein the element and the attribute of the element are selected from at least one of the following list:
- the element is a switch button integrated in a door of a building or of a body of a vehicle, the attribute is the position of the switch button on a structure of the door or on the body of the vehicle, the cognitive data is the time and/or mental load indicative of convenience required by the subject for pushing the switch button to open/close the door and the attribute threshold index is the minimum acceptable time to push the switch button by the subject; and/or
- the element is a textual or graphical indicator on a dashboard of a vehicle for informing the driver about a remaining fuel range or a remaining electric power range, the attribute is the position of the textual or graphical indicator on the dashboard, the cognitive data is the time and/or mental load indicative of anxiety required by the subject for detecting an activation of the textual or graphical indicator and the attribute threshold index is the minimum acceptable time to visually detect the textual or graphical indicator by the subject; and/or
- the element is a tool, knob or other physical instrument used to operate or perform operations on an industry line or inside a vehicle, the attribute is reach and access proximity, the cognitive data is the time and/or placement and/or mental load indicative of stress requirement by the subject for carrying the intended operation and the attribute threshold index is the minimum acceptable time to visually identify and access the instrument by the subject.

8. The method of any of the previous claims, wherein the at least one sensory unit includes one or more of:
- eye tracking camera;
- electrocardiography sensor;
- electroencephalography, EEG, sensor;
- a microphone;
- a touch point tracker;
- a taste sensor;
- a motion detector:
wherein the sensory signals and the sensory response are, respectively:
- position of the eye and point of gaze;
- heart rate and resting heart rate;
- EEG and brain activity indicative of facial expression associated to an emotion, or EEG and brain activity indicative of the cognitive process involved in an action by the subject;
- verbal responses and natural language processing;
- taste perceptions and indicative emotion;
- touch and other physical movements.
- electric signals indicative of a detected movement of the subject inside the environment and data indicative of the position of the subject inside the environment.

9. The method of any of the previous claims, further comprising:
- obtaining personal information pertaining to the subject, the personal information including one or more of demographic data, age, gender, ethnicity, region, country, economic status, education level;
- determining tags to be associated with the subject;
- adjusting the attribute threshold index based on the tags associated with the subject.

10. The method of claim 9, wherein upon determining personal information pertaining to multiple subjects, and upon performing the steps a), b), c), d), e), f) with such multiple steps, the method includes determining behavioral grouping, and recommending trends based on the behavioral grouping of demographic data to further filter recommendations.

11. The method of claims 9, wherein upon determining personal information pertaining to multiple subjects, and upon performing the steps a), b), c), d), e), f) with such multiple steps, the method includes adjusting the attribute threshold index based on the tags associated with the multiple subjects.

12. The method of claim 10, further comprising:
- obtaining personal information pertaining to a new subject, the personal information including one or more of demographic data, age, gender, ethnicity, region, country, economic status, education level,
- determining tags and behavioral grouping associated with the new subject, and
- suggesting a changed attribute of the element of the design feature based on the determined tags and behavioral grouping associated with the new subject.

13. The method of claim 12, further comprises comparing the changed attribute of the element of the design feature with existing regulatory requirements, affecting the changed attribute of the element of the design feature such that the existing regulatory requirements are complied.

14. The method of any of the previous claims, further comprising, before step d): - defining a region of interest by means of at least one sensor, in particular at least one readable tag, more in particular QR codes;
- identifying the region of interest, such that the attribute or changed attribute of the element of the design feature is positioned within the region of interest.

15. The method of claim 14, further comprising identifying an external region of interest which is outside the region of interest, such that the attribute or changed attribute of the element of the design feature is positioned within the external region of interest.
